# EUROPÄISCHE PATENTSCHRIFT

(11) **EP 3 295 857 B1**
(45) Veröffentlichungstag und Bekanntmachung des Hinweises auf die Patenterteilung: **24.07.2019**
(21) Anmeldenummer: 16189419.1
(22) Anmeldetag: 19.09.2016
(51) Int. Cl.: A61B 1/00, A61B 1/12

(54) **ENDOSKOP-TROCKNUNGS- UND LAGERUNGSSYSTEM**
ENDOSCOPE DRYING AND STORAGE SYSTEM
SYSTEME DE STOCKAGE ET DE SECHAGE ENDOSCOPIQUE

(43) Veröffentlichungstag der Anmeldung: 21.03.2018
(73) Patentinhaber: BHT Hygienetechnik GmbH, 86368 Gersthofen (DE)
(72) Erfinder: STUELPNAGEL, Stefan, 86368 Gersthofen (DE)
(74) Vertreter: Schaad, Balass, Menzl & Partner AG

(56) Entgegenhaltungen:
- DE-A1-102009 022 502
- JP-A- 2007 313 113
- US-A1- 2010 145 721
- US-A1- 2010 191 049

## Beschreibung

Die vorliegende Erfindung betrifft ein Verfahren zum Trocknen und Lagern von Endoskopen mit einem Endoskop-Trocknungs- und Lagerungssystem sowie ein Endoskop-Trocknungs- und Lagersystem.

Um chirurgische Geräte und Endoskope nach Gebrauch wieder verwendbar zu machen, ist es notwendig, diese vollständig zu reinigen. Eine solche Reinigung umfasst sämtliche Teile, also auch die inneren Leitungsführungen, Kanäle und Ventilanschlüsse. Nach der rückstandsfreien Reinigung müssen die Geräte desinfiziert und getrocknet werden. Die so aufbereiteten Geräte müssen dann bis zum nächsten Gebrauch keimfrei gelagert und vor Kontamination geschützt werden.

Nach allgemein gültigen Richtlinien (Robert Koch Institut: Anforderungen an die Hygiene bei der Aufbereitung von Medizinprodukten, Anhang 8, Kapitel 3.7) sollen aufbereitete Endoskope in einem Endoskopschrank vorzugsweise arbeitsplatznah aufbewahrt werden. Die Endoskope können dabei in einem einfachen Endoskopschrank, in einem Endoskopschrank mit geregelten Umgebungsbedingungen oder einem Endoskopschrank mit geregelten Umgebungsbedingungen und aktiver Trocknung der Endoskope gelagert werden (DIN EN 16442).

Endoskopschränke mit geregelten Umgebungsbedingungen und aktiver Trocknung der Endoskope erfreuen sich in letzter Zeit einer grossen Beliebtheit. Beispielsweise ist "Olympus Endoscope Drying Cabinet (EDC)" ein Trocken- und Lagerschrank, der speziell für flexible medizinische Endoskope entwickelt wurde. Durch die Verwendung von für den medizinischen Einsatz geeigneter Druckluft im Endoskop-Trockenschrank werden die Endoskope äusserlich getrocknet. Weitere Beispiele sind endoSTORE® vertical von Escad Medical oder die Endoskop-Trockenschränke von Steelco. Diese Trockenschränke können in der Regel 8 bis 16 Endoskope gleichzeitig trocknen und lagern.

WO 2010/136093 betrifft ein modulares Schranksystem zur Trocknung und Aufbewahrung von chirurgischen Instrumenten, insbesondere von Endoskopen, sowie ein Schrankmodul für ein modulares Schranksystem zur Trocknung und Aufbewahrung von chirurgischen Instrumenten, insbesondere Endoskopen.

Gemäss EP 0 986 988 A1 findet das Trocknen der Endoskope in einem Trocknungsschrank statt, der Platz für mehrere Endoskope in ihren jeweiligen Rahmengestellen bietet. In dem Trocknungsschrank wird Trocknungsluft an den Adapter angeschlossen und die Luft durch die inneren Kanäle eines zu trocknenden Endoskops geleitet.

US 2010/0191049 offenbart einen Endoskopspeicherschrank zur Verwendung mit mindestens einem Endoskop, das mindestens eine Alarmfunktion aufweist und eine effiziente Bestandsverwaltung von Endoskopen erlaubt.

Grössere Krankenhäuser verfügen häufig über eine grössere Anzahl Endoskope, zudem fallen die Reinigungen und die für eine allfällige Einlagerung erforderliche Trocknung der Endoskope zu unterschiedlichen Zeitpunkten an. Dies führt dazu, dass die Krankenhäuser häufig mehrere dieser Endoskopschränke mit geregelten Umgebungsbedingungen und aktiver Trocknung besitzen.

Nachteilig an diesen Endoskopschränken mit geregelten Umgebungsbedingungen und aktiver Trocknung sind jedoch die hohen Anschaffungskosten und die erheblichen Unterhaltskosten.

Aufgabe der vorliegenden Erfindung ist es, ein Verfahren zum Trocknen und Lagern von Endoskopen sowie ein Endoskop-Trocknungs- und Lagerungssystem bereitzustellen, das signifikant kostengünstiger und ressourceneffizienter ist, aber eine gleichbleibende Reinhaltequalität sicherstellt.

Die Aufgabe wird durch das Verfahren gemäss Anspruch 1 und den Gegenstand gemäss Anspruch 7 gelöst. Weitere bevorzugte Ausführungsformen sind Gegenstand der abhängigen Ansprüche.

Das erfindungsgemässe Verfahren betrifft ein Verfahren zum Trocknen und Lagern von Endoskopen mit einem Endoskop-Trocknungs- und Lagerungssystem, enthaltend wenigstens zwei Endoskopschränke und eine Datenverarbeitungseinheit, wobei der erste Endoskopschrank von dem zweiten Endoskopschrank verschieden ist, und der erste Endoskopschrank eine aktive Trocknungsfunktion aufweist. Das erfindungsgemässe Verfahren umfasst insgesamt wenigstens sieben Schritte, die nachfolgend detailliert erläutert werden.

In einem ersten Schritt (a) wird ein Endoskop in den ersten Endoskopschrank eingelegt. Das Endoskop wurde vorgängig mit einem handelsüblichen RDG-E-Gerät (Reinigungs- und Desinfektionsgerät für Endoskope) gereinigt und ist noch nass oder feucht. Eine Identifizierungsmarke des Endoskops wird mit einem dem ersten Endoskopschrank zugeordneten Lesegerät eingelesen und die eingelesenen Daten an die Datenverarbeitungseinheit übermittelt, wobei beim Einlesen ein erster Einlesezeitpunkt (E1) ermittelt wird.

In einem zweiten Schritt (b) wird das Endoskop in dem ersten Endoskopschrank getrocknet.

In einem dritten Schritt (c) wird das getrocknete Endoskop aus dem ersten Endoskopschrank entnommen, die Identifizierungsmarke des Endoskops aus dem ersten Endoskopschrank ausgelesen und die ausgelesenen Daten an die Datenverarbeitungseinheit übermittelt, wobei beim Auslesen ein erster Auslesezeitpunkt (A1) ermittelt und erfasst wird.

In einem vierten Schritt (d) wird das getrocknete Endoskop in den zweiten Endoskopschrank eingelegt und die Identifizierungsmarke des Endoskops mit einem dem zweiten Endoskopschrank zugeordneten Lesegerät eingelesen und die eingelesenen Daten an die Datenverarbeitungseinheit übermittelt, wobei beim Einlesen ein zweiter Einlesezeitpunkt (E2) ermittelt und erfasst wird.

In einem fünften Schritt (e) wird in der Datenverarbeitungseinheit überprüft, ob die Differenz des zweiten Einlesezeitpunktes E2 und des ersten Auslesezeitpunktes A1 kleiner als eine vorgegebene maximale Transferdauer tₜᵣₐₙₛ ist.

In einem sechsten Schritt (f) wird wiederholt in der Datenverarbeitungseinheit überprüft, ob die Differenz aus der aktuellen Zeit und dem ersten Einlesezeitpunkt E1 eine vorgegebene maximale Lagerdauer tₘₐₓ unterschreitet. Diese wiederholte Überprüfung findet in regelmässigen Zeitabständen statt. Mindestens einmal täglich findet die Überprüfung statt, vorzugsweise aber wenigstens einmal stündlich.

In einem siebten Schritt (g) wird eine Alarmfunktion ausgelöst, wenn entweder
- wenigstens einer der Werte E1, A1 oder E2 fehlt,
- die maximale Transferdauer tₜᵣₐₙₛ überschritten wird, oder
- die maximale Lagerdauer tₘₐₓ überschritten wird.

Wenn wenigstens einer der Werte E1, A1 oder E2 fehlt bedeutet dies, dass das Endoskop entweder nicht im ersten Endoskopschrank war oder aber nicht sachgemäss ein- oder ausgelesen wurde. Ein solches Endoskop darf aus hygienischen Sicherheitsgründen nicht mehr weiterverwendet werden, weshalb die Alarmfunktion ausgelöst wird. Gleiches gilt für den Wert E2, da hier beispielsweise das Endoskop nicht korrekt eingelesen wurde.

Ebenso wird die Alarmfunktion ausgelöst, wenn die maximale Transferdauer tₜᵣₐₙₛ überschritten wird. Unter der Transferdauer wird diejenige Zeitdauer verstanden, die vom Auslesezeitpunkt A1 aus dem ersten Endoskopschrank bis zum Einlesezeitpunkt E2 im zweiten Endoskopschrank dauert, d.h. das Delta von A1 und E2. So kann sichergestellt werden, dass ein Endoskop nicht zwischen dem Trocknungsschritt und dem Lagerungsschritt unsachgemäss zwischengelagert wird und dadurch kontaminiert werden könnte. Vorzugsweise ist die Transferdauer tₜᵣₐₙₛ weniger als 8 Minuten, besonders bevorzugt weniger als 5 Minuten und idealerweise weniger als 3 Minuten.

Schliesslich wird die Alarmfunktion auch ausgelöst, wenn die maximale Lagerdauer tₘₐₓ überschritten wird. Die maximale Lagerdauer ist in der RKI-Richtlinien "Bundesgesundheitsbl 2012 55:1244-1310,DOI 10.1007/s00103-012-1548-6, insbesondere Kapitel 3.7 und Anlage 8" als Empfehlung festgehalten und beträgt zum gegenwärtigen Zeitpunkt 14 Tage. Wenn die maximale Lagerdauer überschritten wird, muss das Endoskop erneut desinfiziert werden.

Das erfindungsgemässe Verfahren erlaubt damit eine wirtschaftlich effiziente, ressourcenschonende Trocknung und Lagerung von Endoskopen, das gleichzeitig die Sicherheit der Patienten erhöht. Ausserdem können sämtliche Sicherheitsrichtlinien erfüllt werden. Durch die Entkopplung des Trocknungs- und des Lagerungsschrittes kann der sehr kosten- und wartungsintensive Trocknungsschrank für die Trocknung vieler Endoskope verwendet werden und ist nicht mehr aufgrund der Lagerung der Endoskope blockiert. Insbesondere ist es auch möglich, verschiedene Typen von Endoskopen mit dem ersten Endoskopschrank zu trocken. Ausserdem ist es durch die Entkopplung des Trocknungs- und des Lagerungsschrittes einfacher, den ersten und den zweiten Endoskopschrank zu reinigen, zu desinfizieren und zu warten. Gleichzeitig erlaubt das erfindungsgemässe Verfahren auch eine detaillierte Kontrolle über die Lagerdauer des Endoskops. Ausserdem erhält man durch das erfindungsgemässe Verfahren eine bessere Übersicht über den Lagerbestand, was die Bewirtschaftung des Lagers und die Krankenhauslogistik wesentlich vereinfacht.

Vorzugsweise ist der erste Endoskopschrank ein Endoskopschrank mit geregelten Umgebungsbedingungen und einer aktiven Trockenfunktion. Solche Schränke sind dem Fachmann bekannt. Unter einer aktiven Trocknungsfunktion wird in der vorliegenden Erfindung verstanden, dass die Innen- und Aussentrocknung der Endoskope mechanisch unter Zuhilfenahme von beispielsweise Ausblasen mit medizinischer Luft und/oder leicht erhöhten Temperaturen erfolgt. Unter geregelten Umgebungsbedingungen wird verstanden, dass auch nach der aktiven Trocknung das Innenklima des Schranks aktiv beeinflusst wird, indem beispielsweise die zugeführte Luft wohldefinierte Parameter einhält bzgl. mikrobiologische Beschaffenheit, Druck, Temperatur etc.

Sowohl der erste als auch der zweite Endoskopschrank weisen je ein ihm zugeordnetes Lesegerät auf. Dieses Lesegerät kann direkt am Endoskopschrank befestigt sein. Alternativ kann ein mobiles Lesegerät benutzt werden, das den jeweiligen Endoskopschrank erkennt und damit ermöglicht, dass das Lesegerät bei dem Ein- und Ausleseschritt genau dem Endoskopschrank zugeordnet werden kann.

In einer bevorzugten Ausführungsform ist das Lesegerät in der unmittelbaren Umgebung des jeweiligen Endoskopschranks, vorzugsweise an einer Schrankinnen- oder Schrankaussenwand, an der Schranktüre, am Türrahmen des Endoskopschranks oder einer Wandhalterung neben dem Endoskopschrank angeordnet.

Das dem Endoskopschrank zugeordnete Lesegerät kann beispielsweise ein RFID-Lesegerät, ein Barcode-Lesegerät, eine Magnetfelderkennungsvorrichtung oder eine optische Erkennung (Foto, Video) sein. Hierbei ist es aus Kostengründen bevorzugt, wenn nur eine Art der Lesegeräte für die wenigstens zwei Endoskopschränke vorgesehen ist, also entweder ein RFID-Lesegerät, ein Barcodelesegerät oder eine Magnetfelderkennungsvorrichtung. Es können allerdings auch zwei oder mehrere dieser Lesegeräte pro Endoskopschrank vorgesehen sein, um ein Ein- und Auslesen von verschiedenen Endoskopen zu ermöglichen.

Vorzugsweise sind im Lesegerät des ersten und des zweiten Endoskopschranks die Endoskope hinterlegt, die in diesen Schränken getrocknet und gelagert werden dürfen. Dies ermöglicht die Ablehnung des Endoskops, wenn es nicht korrekt identifiziert werden kann.

Vorzugsweise befindet sich eine allfällige Antenne des Lesegeräts an einer Aussenfläche oder in einer Wand des Endoskopschranks, idealerweise auf der Seite, an der die Türe angebracht ist. Wenn das Lesegerät eine RFID-Antenne aufweist, kann diese gewunden, in Kurven, in Schlaufen, mit Verzweigungen und/oder spiralförmig in einer Endoskopschrankwand oder in dessen Schranktüre verlegt sein, so dass der grösste Abstand zwischen der Antenne und dem in der Halterung gehaltenen Aufnahmekorb, in dem das Endoskop während des Trocknungsvorgangs angeordnet sein kann, möglichst klein ist. Die Antenne kann alternativ oder zusätzlich aus zwei oder mehr Antennen bestehen.

Die am Endoskop angebrachte Identifikationsmarke kann beispielsweise ein Funketikett sein und einen Transponder, bevorzugt einen RFID-Chip bzw. einen RFID-Tag, aufweisen. In diesem Fall kann das Lesegerät ein RFID-Lesegerät bzw. ein RFID-Lese- und Schreibgerät sein und eine RFID-Antenne aufweisen. Die RFID-Chips können einen vorzugsweise permanenten Speicher aufweisen, in dem die Produktinformation und ggf. weitere, das Endoskop kennzeichnende Informationen abgespeichert sind, die mithilfe des Lesegeräts ein- und ausgelesen werden können. Die Informationen können in verschlüsselter oder in unverschlüsselter Form gespeichert und/oder übertragen werden. Weitere Details zur Kommunikation zwischen Lesegerät und Identifikationsmarken in RFID-Systemen sind dem Fachmann bekannt.

Die am Endoskop angebrachte Identifikationsmarke kann aber auch eine Barcode-Identifizierungsmarke oder eine Magnetcode-Identifizierungsmarke sein, die mit einem Barcode-Lesegerät oder mit einem Magnetcode-Lesegerät ein- und ausgelesen werden kann. Eine Barcode-Identifikationsmarke kann sowohl eindimensionale als auch mehrdimensionale Barcodes umfassen, wie beispielsweise Strichcodes, Stapelcodes oder Matrixcodes (wie DataMatrix-Codes gemäss ISO/IEC 16022:2006).

Beim Einlesen in den ersten Endoskopschrank wird ein erster Einlesezeitpunkt E1 ermittelt und erfasst. Dieser Einlesezeitpunkt E1 kann entweder durch das Lesegerät oder durch die Datenverarbeitungseinheit ermittelt und erfasst werden. Die Ermittlung des Einlesezeitpunkts ist der Zeitpunkt, an dem das Lesegerät das Einlesen der Identifizierungsmarke registriert oder an dem die Daten des Einlesens bei der Datenverarbeitungseinheit eintreffen. Falls das Lesegerät die Einlesezeit ermittelt und erfasst, anschliessend wird sie an die Datenverarbeitungseinheit übermittelt. Gleiches geschieht mit dem ersten Auslesezeitpunkt A1 am ersten Endoskopschrank und dem zweiten Einlesezeitpunkt E2 am zweiten Endoskopschrank. Die Datenverarbeitungseinheit ordnet die Ein- und Auslesezeitpunkte E1, A1 und E2 immer jeweils einem definierten Endoskop zu, so dass eine Verwechslung der Endoskope ausgeschlossen ist.

Die Alarmfunktion kann optisch und/oder akustisch erfolgen. Bei einer akustischen Alarmfunktion ist beispielsweise eine Alarmfunktion am Schrank selbst oder bei der zentralen Datenverarbeitungseinheit denkbar. Bei einer optischen Alarmfunktion ist beispielsweise ein Blinklicht am Schrank oder eine zu quittierende Mitteilung auf dem Bildschirm der Datenverarbeitungseinheit denkbar.

Alternativ kann das Endoskop, das vorzugsweise in einem Verwaltungsprogramm erfasst ist, für Anwendungen am Patienten gesperrt werden, so dass erst eine erneute Reinigung erfolgen muss.

Nachdem das Endoskop in den ersten Endoskopschrank eingelegt wurde, wird es vorzugsweise über einen Adapter an das Trocknungssystem angehängt. Die für den Trocknungsprozess benötigte Luft wird vorzugsweise mittels eines Filtersystems gereinigt. Die Luft wird vorzugsweise mittels Druck über den Adapter in das Endoskop geleitet und trocknet so das Endoskop von innen. Vorzugsweise wird ein Druck von 0.1 bis 1.0 bar, besonders bevorzugt von 0.4 bis 0.6 bar verwendet. Aussen wird das Endoskop vorzugsweise durch Umluft-Zirkulation getrocknet (DIN EN 16442). Bei einem Luftwechsel von mindestens dem zehnfachen Volumen des Lagerungsraumes in einer Stunde wird eine gute Trocknung erzielt.

Vorzugsweise erfolgt das Einlesen und Auslesen des Endoskops kontaktlos. Das kontaktlose Ein- und Auslesen ermöglicht einen schnellen Transfer vom ersten Endoskopschrank in den zweiten Endoskopschrank. Zudem wird das Risiko einer Beschädigung des Endoskops minimiert, da sich das Personal ausschliesslich auf den Transfer konzentrieren kann.

Erfindungsgemäss weist der zweite Endoskopschrank keine aktive Trocknungsfunktion auf. Da das Endoskop bereits getrocknet in den zweiten Endoskopschrank gelegt wird, ist eine aktive Trocknungsfunktion nicht mehr nötig. Dadurch können erhebliche Kosten gespart werden, da die Trocknungssysteme der Endoskopschränke zum einen teuer in der Anschaffung, aber auch deutlich teurer im Unterhalt sind.

Vorzugsweise wird in der Datenverarbeitungseinheit zusätzlich wiederholt überprüft, ob die Differenz aus der aktuellen Zeit und der ersten Einlesezeit E1 eine vorgegebene Restlagerdauer t_{Rest} unterschreitet. Vorzugsweise ist die Restlagerdauer t_{Rest} 1 bis 2 Tage, besonders bevorzugt 1 Tag, so dass das Endoskop noch an diesem Tag verwendet werden kann. Wenn die Restlagerdauer t_{Rest} des Endoskops einen vorgegebenen Zeitraum unterschreitet, wird eine Alarmfunktion in Form einer Ablauf-Warnung ausgelöst. Durch diese Funktion können erhebliche Kosten gespart werden, da der Benutzer rechtzeitig darauf aufmerksam gemacht wird, wenn ein Endoskop die maximale Lagerdauer bald erreicht hat. Dadurch kann die Auswahl des Endoskops besser gesteuert werden und so verhindert werden, dass die "frischen" Endoskope (Delta von tₐₖₜᵤₑₗₗ- E₁ noch sehr klein und deutlich kleiner als tₘₐₓ) ständig gebraucht werden, während die "älteren" Endoskope (Delta von tₐₖₜᵤₑₗₗ- E₁ schon gross und in der Nähe von tₘₐₓ) im Endoskopschrank liegen bleiben und dadurch wieder erneut aufbereitet werden müssen.

Vorzugsweise ist der Aufnahmekorb, in dem das Endoskop während des Trocknungsvorgangs angeordnet sein kann, sowohl mit dem ersten Endoskopschrank als auch mit dem zweiten Endoskopschrank kompatibel. Dadurch muss das Endoskop beim Transfer vom ersten Endoskopschrank zum zweiten Endoskopschrank nicht oder zumindest kaum berührt werden, sondern der Transfer kann direkt in dem Korb umgesetzt werden.

Alternativ kann das Endoskop während des Trocknungsvorgangs in dem ersten Endoskopschrank und während des Lagerungsvorgangs in dem zweiten Endoskopschrank an einem ausziehbaren Schlitten befestigt werden, sodass das Endoskop bequem vor dem Schrank entnommen werden kann. Ebenso ist eine Kombination von Korb- und Schlittensystem bei den unterschiedlichen Endoskopschränken möglich.

Vorzugsweise weist das Endoskop-Trocknungs- und Lagerungssystem mehr als zwei Endoskopschränke auf. Dabei ist der erste Endoskopschrank von den weiteren Endoskopschränken verschieden und weist eine aktive Trocknungsfunktion auf. Er entspricht dem ersten Endoskopschrank eines oben beschriebenen Endoskop-Trocknungs- und Lagerungssystems, bei dem nur zwei Endoskopschränke beschrieben wurden. Die weiteren Endoskopschränke können voneinander verschieden oder gleich, vorzugsweise gleich sein. Diese sind dazu bestimmt, das getrocknete Endoskop zur Lagerung aufzunehmen. Die Funktion dieser Endoskopschränke ist identisch mit der oben für den zweiten Endoskopschrank erläuterten Funktion, in dem mit einem dem weiteren Endoskopschrank zugeordneten Lesegerät die Identifikationsmarke des Endoskops eingelesen wird, wobei der zweite Einlesezeitpunkt (E2) ermittelt und erfasst wird, der wiederum an die Datenverarbeitungseinheit übermittelt wird.

Vorzugsweise wird das erfindungsgemässe Verfahren mit einem Endoskop-Trocknungs- und Lagerungssystem durchgeführt, das wenigstens zwei Endoskopschränke und eine Datenverarbeitungseinheit enthält, wobei der erste Endoskopschrank von dem zweiten Endoskopschrank verschieden ist, und der erste Endoskopschrank eine aktive Trocknungsfunktion aufweist. Jeder Endoskopschrank weist ein ihm zugeordnetes Lesegerät auf, das wiederum mit der Datenverarbeitungseinheit verbunden ist. Mögliche Lesegeräte sind bereits weiter oben detailliert beschrieben worden.

Erfindungsgemäss weist der zweite Endoskopschrank des Endoskop-Trocknungs- und Lagerungssystem keine aktive Trocknungsfunktion auf, besonders bevorzugt weist er auch keine Vorrichtung auf, um die Umgebungsbedingungen zu regeln. Es hat sich nämlich herausgestellt, dass nach einer gründlichen Trocknung im ersten Endoskopschrank die Regelung der Umgebungsbedingungen nicht mehr nötig ist. Dadurch können hohe Investitionskosten, Wartungs- und Unterhaltskosten vermieden werden.

Weitere Einzelheiten und Vorteile der Erfindung ergeben sich aus den nachfolgenden Zeichnungen.
- Figur 1: zeigt ein exemplarisches Flussdiagramm des erfindungsgemässen Verfahrens.
- Figur 2: zeigt ein Endoskop-Trocknungs- und Lagerungssystem zur Durchführung des erfindungsgemässen Verfahrens.

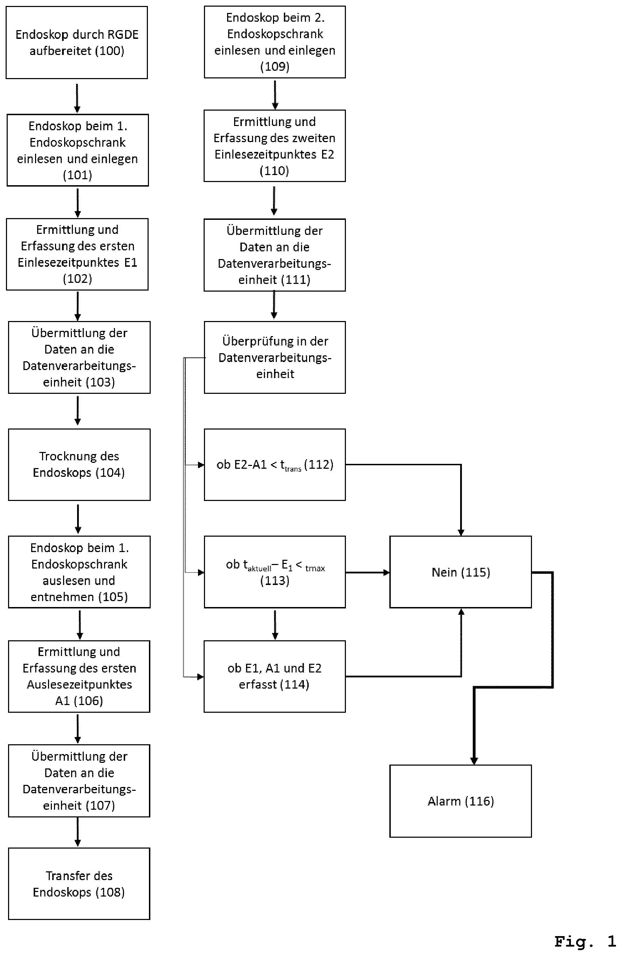

Im Flussdiagramm gemäss Figur 1 ist das erfindungsgemässe Verfahren exemplarisch dargestellt. Zuerst wird ein durch ein Reinigungs- und Desinfektionsgerät RDGE aufbereitetes Endoskop (100) in den ersten Endoskopschrank eingelesen und eingelegt (101). Vorzugsweise muss sich dazu das medizinische Personal am Lesegerät des ersten Endoskopschranks anmelden. Ebenso ist es vorteilhaft, wenn im Lesegerät des ersten Endoskopschranks oder einer damit gekoppelten Steuerung (wie beispielsweise in der Steuerung des ersten Endoskopschranks) das Endoskop hinterlegt ist, sodass die Identität des Endoskops einwandfrei überprüft werden kann. Wenn ein Endoskop nicht erkannt wird, könnte in diesem Fall das Endoskop abgelehnt und eine Trocknung verweigert werden. Beim Einlesen des Endoskops wird der erste Einlesezeitpunkt E1 erfasst (102). Sämtliche eingelesenen und erfassten Daten werden dann an die Datenverarbeitungseinheit (103) übermittelt.

Anschliessend erfolgt die Trocknung des Endoskops mittels einer aktiven Trocknungsfunktion (104). Vorzugsweise erfolgt eine optische Meldung, beispielsweise auf einem Display, wenn das Trocknungsprogramm abgelaufen ist. Sobald das Endoskop getrocknet ist, kann es aus dem ersten Endoskopschrank wieder ausgelesen und entnommen werden (105). Dabei wird der erste Auslesezeitpunkt A1 ermittelt und erfasst (106). Sämtliche ausgelesenen und erfassten Daten werden dann an die Datenverarbeitungseinheit (107) übermittelt.

Anschliessend erfolgt der Transfer des Endoskops (108). Beim zweiten Endoskopschrank angekommen, wird das Endoskop eingelesen und eingelegt (109). Vorzugsweise muss sich dazu das medizinische Personal ebenfalls am Lesegerät des zweiten Endoskopschranks anmelden. Ebenso ist es auch hier vorteilhaft, wenn im Lesegerät des zweiten Endoskopschranks oder einer damit gekoppelten Steuerung (wie beispielsweise in der Steuerung des zweiten Endoskopschranks) das Endoskop hinterlegt ist, sodass die Identität des Endoskops einwandfrei überprüft werden kann. Beim Einlesen des Endoskops wird der zweite Einlesezeitpunkt E2 erfasst (110). Sämtliche eingelesenen und erfassten Daten werden dann an die Datenverarbeitungseinheit (111) übermittelt. Vorzugsweise ist das Lesegerät des zweiten Endoskopschranks und/oder die Datenverarbeitungseinheit, in der Lage festzustellen, wenn das Endoskop nicht korrekt getrocknet wurde.

Anschliessend wird in der Datenverarbeitungseinheit überprüft, ob die Differenz des zweiten Einlesezeitpunktes E2 und des ersten Auslesezeitpunktes A1 kleiner als eine vorgegebene maximale Transferdauer tₜᵣₐₙₛ (112) ist.

Ebenso wird wiederholt in der Datenverarbeitungseinheit überprüft, ob die Differenz aus der aktuellen Zeit tₐₖₜᵤₑₗₗ und dem ersten Einlesezeitpunkt E1 eine vorgegebene maximale Lagerdauer tₘₐₓ unterschreitet (113).

Schliesslich wird noch überprüft, ob sowohl der erste Einlesezeitpunkt E1, der erste Auslesezeitpunkt A1 und der zweite Einlesezeitpunkt E2 erfasst und übermittelt worden sind (114).

Wenn eine der obigen Überprüfungen negativ ausfällt (115), d.h. wenn entweder
- die maximale Transferdauer tₜᵣₐₙₛ überschritten wird,
- wenigstens einer der Werte E1, A1 oder E2 fehlt, oder
- die maximale Lagerdauer tₘₐₓ überschritten wird,
wird ein Alarm ausgelöst.

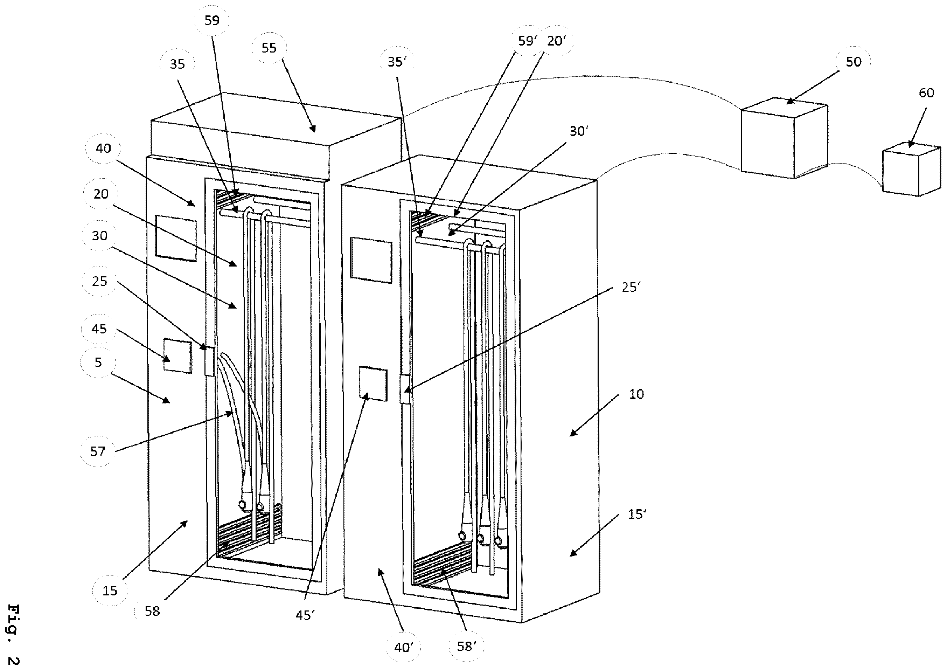

Figur 2 zeigt ein Endoskop-Trocknungs- und Lagerungssystem mit einem ersten Endoskopschrank 5 und einem zweiten Endoskopschrank 10. Beide Endoskopschränke 5 und 10 beinhalten einen wenigstens einseitig offenen Kubus 15, 15', der auf der wenigstens einen offenen Seite 20, 20' mit einer Türe 25, 25' verschliessbar ist. Es ist aber beispielsweise auch denkbar, dass der Kubus an zwei einander gegenüberliegenden Seiten, beispielsweise Vorderseite und gegenüberliegende Rückwand, offen ist und somit eine Durchreichefunktion möglich ist. Der Kubus 15, 15' weist einen Innenraum 30, 30' auf, der Mittel zum Befestigen von Endoskopen 35, 35' aufweist.

Dazu kann beispielsweise an der Schrankdecke zugewandten Seite ein seitlich angebrachter Schlitten mit beispielsweise zwei Vorsprüngen 35, 35' verwendet werden, an denen Endoskope eingehängt werden können. Der Schlitten ist ausziehbar, was ein bequemes Einhängen und Entnehmen der Endoskope ermöglicht. Es sind aber auch Befestigungsmittel denkbar, wie beispielsweise Mittel zum Befestigen von mobilen Körben, in denen die Endoskope gelagert werden können. Solche Systeme sind dem Fachmann bekannt.

Im Randbereich 40, 40' des Kubus ist ein Lesegerät 45, 45' und gegebenenfalls ein Display angebracht, das dazu dient, das Endoskop ein- und auszulesen. Alternativ kann das Lesegerät auch an oder in der Türe angebracht sein. Es ist auch denkbar, dass wenigstens ein mobiles Lesegerät verwendet wird, wobei dieses die Fähigkeit haben muss, einzelne Endoskopschränke voneinander zu unterscheiden. Die Lesegeräte 45 und 45' sind jeweils mit einer Datenverarbeitungseinheit verbunden. Die Verbindung ist vorzugsweise kontaktlos.

Im Innenraum 30 des ersten Endoskopschranks 5 ist ein Trocknungsaggregat 55 angebracht, an das das Endoskop mittels eines Adapters 57 angehängt werden kann, durch den die Luft vorzugsweise mittels Druck in das Endoskop geleitet werden kann, sodass das Endoskop von innen trocknet. Für die äusserliche Trocknung der Endoskope wird beispielsweise über ein Gitter 58, 59 medizinische Druckluft in den Endoskopschrank 5 eingeleitet. Der zweite Endoskopschrank 10 weist kein Trocknungsaggregat auf und hat daher keine aktive Trocknungsfunktion. Allerdings können über die Gitter 58', 59' die Umgebungsbedingungen kontrolliert werden.

Bei der Durchführung des erfindungsgemässen Verfahrens wird das gereinigte und desinfizierte Endoskop mittels einer Identifikationsmarke des Endoskops am Lesegerät 45 des ersten Endoskopschranks 5 eingelesen und am Vorsprung 35 befestigt, sodass das Endoskop im ersten Endoskopschrank eingelegt ist. Beim Einlesen der Identifikationsmarke am Lesegerät 45 übernimmt das Lesegerät vorzugsweise einige relevante Daten des Endoskops, wie beispielsweise eine spezifische Gerätenummer, die letzte Verwendung, Daten der Reinigung und der Desinfektion und ermittelt und erfasst gleichzeitig den Zeitpunkt E1, an dem das Endoskop bei dem Lesegerät 45 des ersten Endoskopschranks 5 eingelesen wurde. Diese Daten werden an eine Datenverarbeitungseinheit 50 mit einer Benutzerschnittstelle 60 übermittelt. Die Benutzerschnittstelle kann beispielsweise ein Display und ein Mittel zum Erfassen von Befehlen durch den Benutzer umfassen.

Das Endoskop wird mit dem Trocknungsaggregat 55 vorzugsweise über einen Adapter 57 verbunden und das Endoskop getrocknet. Nach dem Trocknungsvorgang wird das Endoskop dem ersten Endoskopschrank 5 entnommen und beim Lesegerät 45 ausgelesen. Vorzugsweise werden dabei auch Daten zum Trocknungsvorgang ausgelesen. Gleichzeitig ermittelt und erfasst das Lesegerät 45 den Zeitpunkt A1, an dem das Endoskop bei dem Lesegerät 45 des ersten Endoskopschranks 5 ausgelesen wurde. Diese Daten werden an die Datenverarbeitungseinheit 50 übermittelt.

Anschliessend wird das getrocknete Endoskop mittels der Identifikationsmarke des Endoskops am Lesegerät 45' des zweiten Endoskopschranks 10 eingelesen und am Vorsprung 35' befestigt, sodass das Endoskop im zweiten Endoskopschrank eingelegt ist. Beim Einlesen der Identifikationsmarke am Lesegerät 45' übernimmt das Lesegerät vorzugsweise einige relevante Daten des Endoskops wie beispielsweise eine spezifische Gerätenummer, die letzte Verwendung, Daten der Reinigung, der Desinfektion und der Trocknung und ermittelt und erfasst gleichzeitig den Zeitpunkt E2, an dem das Endoskop bei dem Lesegerät 45' des zweiten Endoskopschranks 10 eingelesen wurde. Diese Daten werden an die Datenverarbeitungseinheit 50 übermittelt.

In der Datenverarbeitungseinheit 50 wird überprüft, ob die Differenz des zweiten Einlesezeitpunktes E2 und des ersten Auslesezeitpunktes A1 kleiner als eine vorgegebene maximale Transferdauer tₜᵣₐₙₛ ist, ob die Differenz aus einer aktuellen Zeit tₐₖₜᵤₑₗₗ und dem ersten Einlesezeitpunkt E1 eine vorgegebene maximale Lagerdauer tₘₐₓ unterschreitet, und ob sowohl der erste Einlesezeitpunkt E1, der erste Auslesezeitpunkt A1 und der zweite Einlesezeitpunkt E2 erfasst und übermittelt worden sind. Wenn eine der obigen Überprüfungen negativ ausfällt, wird ein Alarm ausgelöst. Ein solcher Alarm kann beispielsweise ein zu quittierender Hinweis auf der Benutzerschnittstelle 60 der Datenverarbeitungseinheit 50 sein.

## Patentansprüche

1. Verfahren zum Trocknen und Lagern von Endoskopen mit einem Endoskop-Trocknungs- und Lagerungssystem, enthaltend wenigstens zwei Endoskopschränke (5, 10) und eine Datenverarbeitungseinheit (50), wobei der erste Endoskopschrank (5) von dem zweiten Endoskopschrank (10) verschieden ist, und der erste Endoskopschrank (5) eine aktive Trocknungsfunktion aufweist (55), umfassend folgende Schritte:
(a) Einlegen eines Endoskops in den ersten Endoskopschrank (5) und Einlesen einer Identifizierungsmarke des Endoskops mit einem dem ersten Endoskopschrank zugeordneten Lesegerät und Übermittlung der eingelesenen Daten an die Datenverarbeitungseinheit (50), wobei beim Einlesen ein erster Einlesezeitpunkt (E1) ermittelt und erfasst wird,
(b) Trocknung des Endoskops in dem ersten Endoskopschrank (5),
(c) Entnahme des getrockneten Endoskops aus dem ersten Endoskopschrank (5) und Auslesen der Identifizierungsmarke des Endoskops aus dem ersten Endoskopschrank (5) sowie Übermittlung der ausgelesenen Daten an die Datenverarbeitungseinheit (50), wobei beim Auslesen ein erster Auslesezeitpunkt (A1) ermittelt und erfasst wird,
(d) Einlegen des getrockneten Endoskops in den zweiten Endoskopschrank (10) und Einlesen der Identifizierungsmarke des Endoskops mit einem dem zweiten Endoskopschrank (10) zugeordneten Lesegeräts und Übermittlung der eingelesenen Daten an die Datenverarbeitungseinheit (50), wobei beim Einlesen ein zweiter Einlesezeitpunkt (E2) ermittelt und erfasst wird,
(e) Überprüfung in der Datenverarbeitungseinheit (50), ob die Differenz des zweiten Einlesezeitpunktes E2 und des ersten Auslesezeitpunktes A1 kleiner als eine vorgegebene maximale Transferdauer tₜᵣₐₙₛ ist,
(f) Wiederholte Überprüfung in der Datenverarbeitungseinheit (50), ob die Differenz aus der aktuellen Zeit und dem ersten Einlesezeitpunkt E1 eine vorgegebene maximale Lagerdauer tₘₐₓ unterschreitet,
(g) Auslösen einer Alarmfunktion, wenn entweder
- wenigstens einer der Werte E1, A1 oder E2 fehlt,
- die maximale Transferdauer tₜᵣₐₙₛ überschritten wird, oder
- die maximale Lagerdauer tₘₐₓ überschritten wird.

2. Verfahren gemäss Anspruch 1, **dadurch gekennzeichnet, dass** das Einlesen und Auslesen des Endoskops kontaktlos erfolgt.

3. Verfahren gemäss einem der vorangehenden Ansprüche, **dadurch gekennzeichnet, dass** der zweite Endoskopschrank (10) keine aktive Trocknungsfunktion aufweist.

4. Verfahren gemäss einem der vorangehenden Ansprüche, **dadurch gekennzeichnet, dass** in dem in der Datenverarbeitungseinheit (50) zusätzlich wiederholt überprüft wird,
ob die Differenz aus der aktuellen Zeit und der ersten Einlesezeit E1 eine vorgegebene Restlagerdauer t_{Rest} unterschreitet und
eine Alarmfunktion ausgelöst wird, wenn die Restlagerdauer t_{Rest} überschritten wird.

5. Verfahren gemäss einem der vorangehenden Ansprüche, **dadurch gekennzeichnet, dass** das Endoskop in einem Aufnahmekorb angeordnet ist, der sowohl mit dem ersten Endoskopschrank als auch mit dem zweiten Endoskopschrank kompatibel ist.

6. Verfahren gemäss einem der vorangehenden Ansprüche, **dadurch gekennzeichnet, dass** das Endoskop-Trocknungs- und Lagerungssystem mehr als zwei Endoskopschränke (5,10) aufweist, wobei der erste Endoskopschrank (5) von den weiteren Endoskopschränken (10) verschieden ist und die weiteren Endoskopschränke voneinander verschieden oder gleich, vorzugsweise gleich, sind und dazu bestimmt sind, das getrocknete Endoskop aufzunehmen, wobei eine Identifizierungsmarke des Endoskops mit einem dem weiteren Endoskopschrank zugeordneten Lesegerät eingelesen wird und Übermittlung der eingelesenen Daten an die Datenverarbeitungseinheit (50), wobei beim Einlesen ein zweiter Einlesezeitpunkt (E2) ermittelt und erfasst wird.

7. Endoskop-Trocknungs- und Lagerungssystem zur Durchführung eines Verfahrens gemäss einem der Ansprüche 1 bis 6, enthaltend wenigstens zwei Endoskopschränke (5,10) und eine Datenverarbeitungseinheit (50) die geeignet ist, die Schritte des Verfahrens nach Anspruch 1 bis 6 auszuführen, wobei der erste Endoskopschrank (5) von dem zweiten Endoskopschrank (10) verschieden ist, und der erste Endoskopschrank (5) eine aktive Trocknungsfunktion (55) aufweist, wobei der erste Endoskopschrank (5) ein ihm zugeordnetes Lesegerät (45)aufweist und der zweite Endoskopschrank (10) ein ihm zugeordnetes Lesegerät (45') aufweist und jedes Lesegerät (45, 45') mit der Datenverarbeitungseinheit (50) verbunden ist, wobei der wenigstens zweite Endoskopschrank (10) keine aktive Trocknungsfunktion (55) aufweist.

8. Endoskop-Trocknungs- und Lagerungssystem gemäss Anspruch 7, **dadurch gekennzeichnet, dass** der wenigstens zweite Endoskopschrank (10) keine Mittel zur Regelung der Umgebungsbedingungen aufweist.

## Claims

1. A method for drying and storing endoscopes with an endoscope drying and storing system, containing at least two endoscope cabinets (5, 10) and a data processing unit (50), wherein the first endoscope cabinet (5) is different from the second endoscope cabinet (10) and the first endoscope cabinet (5) comprises an active drying function (55), comprising the following steps:
(a) introduction of an endoscope into the first endoscope cabinet (5) and reading-in of an identification mark of the endoscope by means of a reading device associated with the first endoscope, and transmission of the read-in data to the data processing unit (50), wherein, during the reading-in, a first reading-in time (E1) is determined and acquired,
(b) drying of the endoscope in the first endoscope cabinet (5),
(c) removal of the dried endoscope from the first endoscope cabinet (5) and reading-out of the identification mark of the endoscope from the first endoscope cabinet (5) as well as transmission of the read-out data to the data processing unit (50), wherein, during the reading-out, a first reading-out time (A1) is determined and acquired,
(d) introduction of the dried endoscope into the second endoscope cabinet (10) and reading-in of the identification mark of the endoscope by means of a reading device associated with the second endoscope cabinet (10), and transmission of the read-in data to the data processing unit (50), wherein, during the reading-in, a second reading-in time (E2) is determined and acquired,
(e) verification in the data processing unit (50) as to whether the difference between the second reading-in time E2 and the first reading-out time A1 is less than a predetermined maximum transfer duration tₜᵣₐₙₛ,
(f) repeated verification in the data processing unit (50) as to whether the difference between the current time and the first reading-in time E1 is below a predetermined maximum storage duration tₘₐₓ,
(g) triggering of an alarm function when
- at least one of the values E1, A1 or E2 is absent, or
- the maximum transfer duration tₜᵣₐₙₛ has been exceeded, or
- the maximum storage duration tₘₐₓ has been exceeded.

2. The method according to Claim 1, **characterized in that** the reading-in and reading-out of the endoscope occur in a contactless manner.

3. The method according to any one of the preceding claims, **characterized in that** the second endoscope cabinet (10) has no active drying function.

4. The method according to any one of the preceding claims, **characterized in that**, in the in the data processing unit (50), it is verified in addition repeatedly
whether the difference between the current time and the first reading-in time E1 is below a predetermined remaining storage duration t_{Rest}, and
an alarm function is triggered when the remaining storage duration t_{Rest} has been exceeded.

5. The method according to any one of the preceding claims, **characterized in that** the endoscope is arranged in a receiving basket which is compatible both with the first endoscope cabinet and with the second endoscope cabinet.

6. The method according to any one of the preceding claims, **characterized in that** the endoscope drying and storing system comprises more than two endoscope cabinets (5, 10), wherein the first endoscope cabinet (5) is different from the additional endoscope cabinets (10) and the additional endoscope cabinets are different from one another or identical to one another, preferably identical to one another, and are intended to receive the dried endoscope, wherein an identification mark of the endoscope is read in by means of a reading device associated with the additional endoscope cabinet, and transmission of the read-in data to the data processing unit (50), wherein, during the reading-in, a second reading-in time (E2) is determined and acquired.

7. An endoscope drying and storing system for carrying out a method according to any one of Claims 1 to 6, containing at least two endoscope cabinets (5, 10) and a data processing unit (50) which is suitable for carrying out the steps of the method according to Claims 1 to 6, wherein the first endoscope cabinet (5) is different from the second endoscope cabinet (10), and the first endoscope cabinet (5) has an active drying function (55), wherein the first endoscope cabinet (5) comprises a reading device (45) associated with same, and the second endoscope cabinet (10) comprises a reading device (45') associated with same, and each reading device (45, 45') is connected to the data processing unit (50), wherein the at least second endoscope cabinet (10) has no active drying function (55).

8. The endoscope drying and storing system according to Claim 7, **characterized in that** the at least second endoscope cabinet (10) has no means for the regulation of the ambient conditions.

## Revendications

1. Procédé de séchage et de stockage d'endoscopes avec un système de séchage et de stockage endoscopique, contenant au moins deux armoires pour endoscope (5, 10) et une unité de traitement de données (50), dans lequel la première armoire pour endoscope (5) est différente de la deuxième armoire pour endoscope (10), et la première armoire pour endoscope (5) présente une fonction de séchage active (55), comprenant les étapes suivantes :
(a) insertion d'un endoscope dans la première armoire pour endoscope (5) et lecture d'une marque d'identification de l'endoscope avec un lecteur associé à la première armoire pour endoscope et transmission des données lues à l'unité de traitement de données (50), dans lequel lors de la lecture un premier moment de lecture (E1) est déterminé et détecté,
(b) séchage de l'endoscope dans la première armoire pour endoscope (5),
(c) prélèvement de l'endoscope séché de la première armoire pour endoscope (5) et relevé de la marque d'identification de l'endoscope de la première armoire pour endoscope (5) ainsi que transmission des données relevées à l'unité de traitement de données (50), dans lequel lors du relevé un premier moment de relevé (A1) est déterminé et détecté,
(d) insertion de l'endoscope séché dans la deuxième armoire pour endoscope (10) et lecture de la marque d'identification de l'endoscope avec un lecteur associé à la deuxième armoire pour endoscope (10) et transmission des données lues à l'unité de traitement de données (50), dans lequel lors de la lecture un deuxième moment de lecture (E2) est déterminé et détecté,
(e) vérification dans l'unité de traitement de données (50), si la différence entre le deuxième moment de lecture E2 et le premier moment de relevé A1 est inférieure à une durée de transfert maximum prédéfinie tₜᵣₐₙₛ,
(f) vérification répétée dans l'unité de traitement de données (50) pour savoir si la différence entre le temps actuel et le premier moment de lecture E1 sous-passe une durée de stockage maximum prédéfinie tₘₐₓ,
(g) déclenchement d'une fonction d'alarme, lorsque soit
- au moins une des valeurs E1, A1 ou E2 manque,
- la durée de transfert maximum tₜᵣₐₙₛ est dépassée, ou
- la durée de stockage maximum tₘₐₓ est dépassée.

2. Procédé selon la revendication 1, **caractérisé en ce que** la lecture et le relevé de l'endoscope se font sans contact.

3. Procédé selon l'une quelconque des revendications précédentes, **caractérisé en ce que** la deuxième armoire pour endoscope (10) ne présente pas de fonction de séchage active.

4. Procédé selon l'une quelconque des revendications précédentes, **caractérisé en ce que** dans lequel il est vérifié de manière répétée en plus dans l'unité de traitement de données (50),
si la différence entre le temps actuel et le premier temps de lecture E1 sous-passe une durée de stockage résiduelle prédéfinie t_{Rest} et
une fonction d'alarme est déclenchée, lorsque la durée de stockage résiduelle t_{Rest} est dépassée.

5. Procédé selon l'une quelconque des revendications précédentes, **caractérisé en ce que** l'endoscope est agencé dans un panier de réception, qui est compatible aussi bien avec la première armoire pour endoscope qu'avec la deuxième armoire pour endoscope.

6. Procédé selon l'une quelconque des revendications précédentes, **caractérisé en ce que** le système de séchage et de stockage endoscopique présente plus de deux armoires pour endoscope (5, 10), dans lequel la première armoire pour endoscope (5) est différente des autres armoires pour endoscope (10) et les autres armoires pour endoscope sont différentes les unes des autres ou les mêmes, de préférence les mêmes et sont destinées à recevoir l'endoscope séché, dans lequel une marque d'identification de l'endoscope est lue avec un lecteur associé à l'autre armoire pour endoscope et transmission des données lues à l'unité de traitement de données (50), dans lequel lors de la lecture un deuxième moment de lecture (E2) est déterminé et détecté.

7. Système de séchage et de stockage endoscopique pour la réalisation d'un procédé selon l'une quelconque des revendications 1 à 6, contenant au moins deux armoires pour endoscope (5, 10) et une unité de traitement de données (50) qui est adaptée pour réaliser les étapes du procédé selon la revendication 1 à 6,
dans lequel la première armoire pour endoscope (5) est différente de la deuxième armoire pour endoscope (10), et la première armoire pour endoscope (5) présente une fonction de séchage active (55), dans lequel la première armoire pour endoscope (5) présente un lecteur (45) qui lui est associé et la deuxième armoire pour endoscope (10) présente un lecteur (45') qui lui est associé et chaque lecteur (45, 45') est relié à l'unité de traitement de données (50),
dans lequel l'au moins deuxième armoire pour endoscope (10) ne présente pas de fonction de séchage active (55).

8. Système de séchage et de stockage endoscopique selon la revendication 7, **caractérisé en ce que** l'au moins deuxième armoire de séchage pour endoscope (10) ne présente pas de moyens de régulation des conditions ambiantes.
